# EUROPÄISCHE PATENTSCHRIFT

(11) **EP 1 524 985 B1**
(45) Veröffentlichungstag und Bekanntmachung des Hinweises auf die Patenterteilung: **19.09.2007**
(21) Anmeldenummer: 03766198.0
(22) Anmeldetag: 17.07.2003
(51) Int. Cl.: A61K 31/7088

(54) **KOSMETISCHE ODER PHARMAZEUTISCHE ZUBEREITUNGEN ENTHALTEND NUKLEINSÄUREN AUF DER BASIS NICHT-METHYLIERTER CPG-MOTIVE**
COSMETIC OR PHARMACEUTICAL PREPARATIONS COMPRISING NUCLEIC ACIDS BASED ON NON-METHYLATED CPG MOTIFS
PREPARATIONS COSMETIQUES OU PHARMACEUTIQUES CONTENANT DES ACIDES NUCLEIQUES A BASE DE MOTIFS CPG NON METHYLES

(30) Priorität: 25.07.2002 DE 10233994
(43) Veröffentlichungstag der Anmeldung: 27.04.2005
(73) Patentinhaber: Phenion GmbH & Co KG, 60439 Frankfurt (DE)
(72) Erfinder: KIPPENBERGER, Stefan, 60316 Frankfurt am Main (DE); BERND, August, 55411 Bingen-Kempten (DE); KAUFMANN, Roland, 63073 Offenbach (DE)
(74) Vertreter: Kluschanzoff, Harald
(86) Internationale Anmeldenummer: PCT/EP2003/007748
(87) Internationale Veröffentlichungsnummer: WO 2004/012688

(56) Entgegenhaltungen:
- WO-A-96/01636
- WO-A-99/60167
- HARTMANN G. ET AL.,: "delineation of a CpG phosphorothioate oligodeoxynucleotide for activating promate immune responses in vitro and in vivo" J. IMMUNOL., Bd. 164, Nr. 3, 1. Februar 2000 (2000-02-01), Seiten 1617-1624, XP002267233
- BALLAS Z.K. ET AL.,: "divergent therapeutic and immunologic effects of oilgodeoxynucleotides with distinct CpG motifs" J. IMMUNOL., Bd. 167, Nr. 9, 1. November 2001 (2001-11-01), Seiten 4878-4886, XP002267234 in der Anmeldung erwähnt
- KRIEG A M ET AL: "GPG MOTIFS IN BACTERIAL DNA TRIGGER DIRECT B-CELL ACTIVATION" NATURE, MACMILLAN JOURNALS LTD. LONDON, GB, Bd. 374, 6. April 1995 (1995-04-06), Seiten 546-549, XP002913424 ISSN: 0028-0836

### SEQUENCE LISTING (Sequenzprotokoll)

<110> Phenion GmbH & Co. KG
<120> Kosmetische oder pharmazeutische Zubereitungen enthaltend Nukleinsäuren auf der Basis nicht-methylierter CpG-Motive
<130> H 5623 PCT
<150> 102 33 994.5-41
   <151> 2002-07-25
<160> 17
<170> SeqWin99, version 1.02
<210> 1
   <211> 20
   <212> DNA
   <213> Artificial Sequence
<220>
   <223> CpG
<400> 1
   tccatgacgt tcctgacgtt 20
<210> 2
   <211> 6
   <212> DNA
   <213> Artificial Sequence
<220>
   <223> CpG
<400> 2
   gacgtt 6
<210> 3
   <211> 8
   <212> DNA
   <213> Artificial Sequence
<220>
   <223> CpG
<400> 3
   tgacgttc 8
<210> 4
   <211> 10
   <212> DNA
   <213> Artificial Sequence
<220>
   <223> CpG
<400> 4
   atgacgttcc 10
<210> 5
   <211> 12
   <212> DNA
   <213> Artificial Sequence
<220>
   <223> CpG
<400> 5
   catgacgttc ct 12
<210> 6
   <211> 14
   <212> DNA
   <213> Artificial Sequence
<220>
   <223> CpG
<400> 6
   ccatgacgtt cctg 14
<210> 7
   <211> 16
   <212> DNA
   <213> Artificial Sequence
<220>
   <223> CpG
<400> 7
   tccatgacgt tcctga 16
<210> 8
   <211> 16
   <212> DNA
   <213> Artificial Sequence
<220>
   <223> CpG
<400> 8
   tcctcaacgt tcctga 16
<210> 9
   <211> 16
   <212> DNA
   <213> Artificial Sequence
<220>
   <223> CpG
<400> 9
   tccgcaacgt tcctga 16
<210> 10
   <211> 16
   <212> DNA
   <213> Artificial Sequence
<220>
   <223> CpG
<400> 10
   tcctcgacgt ccctga 16
<210> 11
   <211> 16
   <212> DNA
   <213> Artificial Sequence
<220>
   <223> CpG
<400> 11
   tcctcagcgc tcctga 16
<210> 12
   <211> 16
   <212> DNA
   <213> Artificial Sequence
<220>
   <223> CpG
<400> 12
   tcctcaacgc tcctga 16
<210> 13
   <211> 16
   <212> DNA
   <213> Artificial Sequence
<220>
   <223> CpG
<400> 13
   tcctcatcga tcctga 16
<210> 14
   <211> 16
   <212> DNA
   <213> Artificial Sequence
<220>
   <223> CpG
<400> 14
   tcctcttcga acctga 16
<210> 15
   <211> 17
   <212> DNA
   <213> Artificial Sequence
<220>
   <223> CpG
<400> 15
   tccatgacgt tcctgac 17
<210> 16
   <211> 18
   <212> DNA
   <213> Artificial Sequence
<220>
   <223> CpG
<400> 16
   tccatgacgt tcctgacg 18
<210> 17
   <211> 19
   <212> DNA
   <213> Artificial Sequence
<220>
   <223> CpG
<400> 17
   tccatgacgt tcctgacgt 19

## Beschreibung

Die vorliegende Erfindung betrifft kosmetische oder pharmazeutische Zubereitungen zur Prophylaxe und/oder Behandlung epithelialen Deckgewebes, die Nukleinsäuren auf der Basis nicht-methylierter CpG-Motive enthalten, die Verwendung solcher Nukleinsäuren auf der Basis nicht-methylierter CpG-Motive zur Prophylaxe und/oder Behandlung epithelialen Deckgewebes sowie Wäscheweichspüler, Handwaschmittel, Körper- und Haarpflegemittel, Haarfärbemittel oder Handgeschirrspülmittel, enthaltend solche Nukleinsäuren auf der Basis nicht-methylierter CpG-Motive.

Unmethylierte CG-reiche DNA-Sequenzen (CpG) sind weit verbreitet im bakteriellen Genom, während sie im Säugergenom deutlich seltener vorkommen.

Hinweise für immunstimulatorische Wirkungen von "Fremd"-DNA gibt es bereits seit den 60er Jahren (Jensen, K. E., Neal, A. L., Owens, R. E. und Warren, J. Interferon Responses of Chick Embryo Fibroblasts to Nucleic Acids and Related Compounds Nature 200 (1963) 433 - 434). Beschrieben wurde sowohl die Induktion der Produktion von Interferon Gamma, als auch die Aktivierung von natürlichen Killerzellen sowie Induktion einer antitumoralen Aktivität durch Fraktionen des Bacillus Calmette-Guerin (BCG) (Tokunaga, T., Yamamoto, H., Shimada, S., Abe, H., Fukuda, T., Fujisawa, Y., Furutani, Y., Yano, O., Kataoka, T., Sudo, T., Makiguchi, N. und Suganuma, T. Antitumor Activity of Deoxyribonucleic Acid Fraction From Mycobacterium Bovis BCG. I. Isolation, Physicochemical Characterizatiori and Antitumor Activity J. Natl. Cancer Res. 72 (1984) 955 - 962). Die immunstimulierende Aktivität dieser Fraktion konnte durch Vorinkubation mit DNAsen, nicht aber mit RNAsen zerstört werden. Dies legte den Schluß nahe, daß die bakterielle DNA den immunstimulierenden Anteil der BCG-Fraktion ausmacht. Die genaue Untersuchung der immunologisch aktiven DNA-Sequenzen ergab, daß es sich dabei um Oligonukleotide handelt, die aus einem zentralen Palindrom mit einem CpG-Motiv bestehen.

Weitere Untersuchungen gaben Anlaß zu der Hypothese, daß sich das für die immunstimulatorische Wirkung wichtige Motiv aus einer zentralen CpG-Gruppe zusammensetzt, die am 5'-Ende von zwei Purinen und am 3'-Ende von zwei Pyrimidinen flankiert wird (Krieg, A. M., Yi, A., Matson, S., Waldschmidt, T. J., Bishop, G. A., Teasdale, R., Koretzky, G. A. und Klinman, D. M. CpG Motifs in Bacterial DNA Trigger direct B-Cell Activation Nature 374 (1995) 546 - 549). CpG-Dinukleotide werden in eukaryontischer DNA supprimiert.

Einerseits kommen diese Motive in eukaryontischer DNA nur mit einem Fünftel der erwarteten Frequenz vor, andererseits sind sie zu 60 - 90 % methyliert (Bird, A. P. CpG-rich Islands and the Function of DNA Methylation Nature 321 (1986) 209 - 213). Im Gegensatz dazu findet man das CpG-Motiv in bakterieller DNA unmethyliert und mit der erwarteten Frequenz (1:16). Es konnte gezeigt werden, daß die Methylierung das stimulatorische Potential des CpG-Motivs zerstört (Krieg, siehe oben). Diese Unterschiede zwischen bakterieller und eukaryontischer DNA ermöglichen es, die biologischen Beobachtungen bezüglich der immunstimulatorischen Wirkung von bakterieller DNA und synthetischen CpG-Oligodeoxynukleotiden (ODN) sinnvoll zu interpretieren.

Die Erkennung von "Fremd"-DNA und die darauf folgende immunologische Reaktion ist für das angeborene Immunsystem eine Möglichkeit zwischen "Selbst" und "Fremd" zu unterscheiden, ohne auf Vermittlung und Intervention des adaptiven Immunsystems angewiesen zu sein.

Die Effekte und der Wirkungsmechanismus der CpG-ODN wurden vor allem im murinen System untersucht. ODN wirken sowohl auf das angeborene als auch auf das adaptive Immunsystem der Maus stimulatorisch, wobei sich die Wirkungen im Bezug auf die Signalübertragung und die Sequenzspezifität unterscheiden.

Es konnte beispielsweise gezeigt werden, daß CpG-ODN auf die unterschiedlichen Typen von Antigen präsentierenden Zellen (APZ) immunstimulatorisch wirken. Die Stimulation von gereinigten B-Lymphozyten mit unmethylierten CpG-ODN führt zur Proliferation und Sekretion von Immunglobulinen (Krieg, siehe oben).

CpG-ODN induzieren in Makrophagen die Aktivierung des Transkriptionsfaktors Nuclear Factor kB (NF-kB), die Transkription von Zytokin-mRNA und die Sekretion von Zytokinen wie TNFa, IL-1, IL-6 und IL-12 (Sparwasser, T., Miethke, T. und Lipford, G. B. Makrophages Sense Pathogens via DNA Motifs: Induction of Tumor Necrosis Factor-Alpha- Mediated Shock Eur. J. Immunol. 27 (1997) 1671-9679).

CpG-ODN wirken sowohl auf reife als auch auf unreife dentritische Zellen aktivierend. Sie verstärken auf beiden Zellpopulationen die MHC II-Expression und die Expression von kostimulatorischen Molekülen (CD40, CD86) und induzieren die Produktion von Zytokinen wie IL-6, IL-12 und TNFa. Um den Wirkmechanismus der CpG-ODN näher zu untersuchen, wurden Versuche mit immobilisierten CpG-ODN durchgeführt. Die Ergebnisse dieser Versuche weisen daraufhin, daß eine Aufnahme der CpG-ODN in die Zelle für die immunstimulatorische Wirkung notwendig ist (Krieg, siehe oben).

Andere Versuche zeigen, daß die Aufnahme der DNA an der Zelloberfläche von Makrophagen durch jedes beliebige kompetitiv zugegebene ODN blockiert werden kann (Häcker, H., Mischak, H., Miethke, T., Liptary, S., Schmid, R., Sparwasser, T., Heeg, K., Lipford, G. B. und Wagner, H. CpG-DNA-Specfic Activation of Antigen-Presenting Cells Requires Stress Kinase Activity and Is Preceded by Non-Specfic Endocytosis and Endosomal Maturation EMBO Journal 17 (1998) 6230-6240), was vermuten läßt, daß die Aufnahme der ODN in die Zelle nicht sequenzspezifisch erfolgt. Dagegen könnte die CpG-Spezifität durch einen intrazellulären Rezeptor, der z.B. im Endosom lokalisiert ist, vermittelt werden.

Die Hypothese der endosomalen Lokalisation eines intrazelluären CpG-Rezeptors wird durch Ergebnisse untermauert, die zeigen, daß die endosomale Ansäuerung für den Signalweg der CpG-ODN notwendig ist, da die CpG-ODN-Wirkung durch Inhibitoren der endosomalen Ansäuerung wie z.B. Chloroquin blockiert wird (Häcker, siehe oben).

Der vollständige Wirkmechanismus und Signalweg der CpG-ODN ist jedoch größtenteils noch ungeklärt. CpG-ODN entfalten ihre Wirkung auf Makrophagen und dentritische Zellen direkt, während die CpG-ODN-Wirkung auf die B-Zellen sowohl direkt als auch im Sinne einer Kostimulation möglich ist (Krieg, siehe oben). Dagegen konnten direkte Wirkungen von CpG-ODN auf T-Zellen nicht gezeigt werden. Jedoch werden T-Zellen, die über eine T-Zellrezeptor-Ligation ihr Signal 1 erhalten, durch CpG-ODN kostimuliert (Bendigs, S., Salzer, U., Lipford, G. B., Wagner, H. und Heeg, K. CpG-Oligodeoxynucleotides Co-Stimulate Primary T Cells in the Absence of Antigen-Presenting Cells Eur. J. Immunol. 29 (1999) 1209-1218).

Diese Ergebnisse lassen vermuten, daß der Mechanismus der T-Zellkostimulation ein anderer ist als der einer direkten CpG-ODN-Wirkung auf APZ. Die Untersuchungen der direkten Wirkung von ODN auf T-Zellen wurden in vitro durchgeführt. Es liegen allerdings viele in vivo-Ergebnisse vor, in denen CpG-ODN als Adjuvans verwendet wurden, und durch die Injektion von CpG-ODN in vivo eine T-Zellaktivierung induziert wurde. Die CpG-ODN unterstützen die Ausbildung einer TH1-Immunantwort und induzieren eine starke peptidspezifische zytotoxische T-Lymphozytenaktivität (ZTL).

Im Gegensatz zum murinen System liegen für die Wirkung von ODN im humanen System nur wenige Befunde vor. Es ist bekannt, daß ODN mit CpG-Motiven in Lymphozyten aus dem peripheren Blut die Produktion von IFNa induzieren. Ferner ist gezeigt, daß durch CpG-ODN - ähnlich wie im murinen System - natürliche Killerzellen durch die Vermittlung von IL-12, das von aktivierten Makrophagen produziert wird, aktiviert werden (Ballas, Z. K., Rasmussen, W. L. und Krieg, A. M. Induction of NK Activity in Murine and Human Cells by CpG Motifs in Oligodeoxynucleotides and Bacterial DNA J. Immunol. 157 (1996) 1840 -1845). Humane periphere mononukleäre Zellen (Peripherblut monocytische Zellen = PBMZ) werden durch CpG-ODN sequenzspezifisch aktiviert.

Diese Aktivierung führt zu einer erhöhten Expression von CD86, CD40, MHC I- und MHC II-Molekülen und zu einer Produktion der Zytokine IL-12, IL-6 und TNFa. Wie im murinen System induzieren CpG-ODN die Proliferation humaner B-Zellen ( Bauer, M., Heeg, K., Wagner, H. und Lipford G. B. DNA Activates Human Immune Cells through a CpG Sequence-Dependent Manner Immunology, 97 (1999) 699 - 705). Die bisher gewonnenen Ergebnisse im humanen System weisen viele Gemeinsamkeiten mit den Ergebnissen im murinen System auf.

CpGs wird daher im Stand der Technik eine immunoprotektive und immunstimulierende Wirkung zugeschrieben und in diesem Sinne werden sie auch - momentan noch experimentell - eingesetzt. Ein suppressiver Effekt von CpGs auf das Immunsystem, insbesondere auf das Immunsystem der Haut war bisher hingegen nicht bekannt.

Überraschenderweise wurde gefunden, daß CpGs bei topischer Anwendung auf der Haut einen immunsuppressiven Effekt ausüben. Speziell die Expression von pro-inflammatorischen Zytokinen bzw. Chemokinen (IL-6 und IL-8) in Hautzellen wird durch Verwendung von CpGs supprimiert.

Die WO 96/01636 offenbart die Verwendung von Oligonukleotiden mit CpG-Motif zur Behandlung von entzündlichen Hauterkrankungen. Die WO 99/60167 beschreibt die Verwendung von anti-sense Oligonukleotiden zur topischen Applikation für dermales und epidermales Gewebe.

Entzündungen des Hautorgans sind weit verbreitete Krankheiten, die sowohl endogen als, auch exogen getriggert sein können. Zur Behandlung werden meist Therapeutika auf Steroidbasis topisch oder systemisch verabreicht. Diese Substanzklasse zeigt neben ihrer Wirksamkeit auch eine Reihe von unerwünschten Nebenwirkungen (z.B. Hautatrophie, Cushing Syndrom). Ein alternatives Prinzip zur Behandlung von Entzündungen der Haut ist daher wünschenswert.

Gegenstand der vorliegenden Erfindung ist daher eine kosmetische oder pharmazeutische Zubereitung zur Prophylaxe und/oder Behandlung epithelialen Deckgewebes, insbesondere zur Prophylaxe und/oder Behandlung entzündlich veränderten epithelialen Deckgewebes, die dadurch gekennzeichnet ist, daß sie Nukleinsäuren auf der Basis nicht-methylierter CpG-Motive enthält.

Unter Nukleinsäuren auf der Basis nicht-methylierter CpG-Motive sind Nukleinsäuren zu verstehen, die mindestens ein nicht-methyliertes zentrales CG-Dinukleotid umfassen. Vorzugsweise, wird das nicht-methylierte CG-Dinukleotid am 5'-Ende von zwei Purinen (Pu), 3'-seitig von zwei Pyrimidinen (Pyr) flankiert. Besonders bevorzugt umfassen erfindungsgemäß einsetzbare Nukleinsäuren auf der Basis nicht-methylierter CpG-Motive mindestens eine Sequenz, die zu 80 % bis 100 %, vorzugsweise zu 85 % bis 100 %, insbesondere zu 90 % bis 100 %, besonders bevorzugt zu 95 % bis 100 % und ganz besonders bevorzugt zu 100 % der Consensus-Sequenz 5'-A/GA/GCGC/TC/T-3' entspricht, wie von Jakob et al., J lmunology, 1998,161:3042-49, beschrieben.

Erfindungsgemäß geeignete Nukleinsäuren auf der Basis nicht-methylierter CpG-Motive weisen eine Länge von 6 bis 40, insbesondere 14 bis 40, vorzugsweise 14 bis 30 bevorzugt 14 bis 25 und ganz besonders bevorzugt von 14 bis 20 Nukleotiden auf.

Besonders geeignete Nukleinsäuren sind beispielsweise im Sequenzprotokoll der WO 01/32877 aufgeführt, auf die hiermit vollumfänglich Bezug genommen wird.

Ganz besonders geeignet sind Nukleinsäuren auf der Basis nicht-methylierter CpG-Motive, die eine Sequenz umfassen, die ausgewählt ist unter

| | |
|---|---|
| CpG-1-PTO: | 5'-TCC ATG ACG TTC CTG ACG TT-3' |
| CpG-9-PTO: | 5'-G ACG TT-3' |
| CpG-10-PTO: | 5'-TG ACG TTC-3' |
| CpG-11-PTO: | 5'-ATG ACG TTC C-3' |
| CpG-12-PTO: | 5'-C ATG ACG TTC CT-3' |
| CpG-13-PTO: | 5'-CC ATG ACG TTC CTG-3' |
| CpG-14-PTO: | 5'-TCC ATG ACG TTC CTG A-3' |
| CpG-14A-PTO: | 5'-TCC TCA ACG TTC CTG A-3' |
| CpG-14B-PTO: | 5'-TCC GCA ACG TTC CTG A-3' |
| CpG-14C-PTO: | 5'-TCC TCG ACG TCC CTG A-3' |
| CpG-14D-PTO: | 5'-TCC TCA GCG CTC CTG A-3' |
| CpG-14E-PTO: | 5'-TCC TCA ACG CTC CTG A-3' |
| CpG-14F-PTO: | 5'-TCC TCA TCG ATC CTG A-3' |
| CpG-14G-PTO: | 5'-TCC TCT TCG AAC CTG A-3' |
| CpG-15-PTO: | 5'-TCC ATG ACG TTC CTG AC-3' |
| CpG-16-PTO: | 5'-TCC ATG ACG TTC CTG ACG-3' |
| CpG-17-PTO: | 5'-TCC ATG ACG TTC CTG ACG T-3' |

Die erfindungsgemäß einsetzbaren Nukleinsäuren auf der Basis nicht-methylierter CpG-Motive können in dem Fachmann bekannter Weise vollständig (alle Nukleotide) oder teilweise (nur einige Nukleotide) chemisch modifiziert sein. Bevorzugte Modifikationen sind beispielsweise:
a) Veränderung der Internucleosidbrücken: Austausch von Phosphodiestern gegen Methylphosphonate, Phosphoramidate, Phosphorothioate oder Hydroxylamine;
b) Veränderung der Zuckerkomponenten: Austausch der Ribose gegen diverse Hexo- bzw. Pentopyranosen oder 3'-5'-carbocyclisch verbrückte Derivate der 2'-Deoxyribose (Steffens R & Leumann CJ (1997) Tricyclo-DNA: A phosphodiesterbackbone based DNA analog exhibiting strong comlementary base-pairing properties. J. Am. Chem. Soc. 119, 11548-11549);
c) Austausch des Strangrückgrats: Austausch der Polyesterketten auf Basis von Zucker-Phosphat-Einheiten gegen Carboxamidketten auf Basis von Aminosäurederivaten wie N-(2-Aminoethyl)-glycin-Einheiten

Erfindungsgemäß besonders bevorzugt sind Hybridmoleküle, die aus CpG-Haltigen DNAIRNA-Sequenzen bestehen.

Im Rahmen der vorliegenden Erfindung wird unter epithelialem Deckgewebe zum einen die die äußere Körperoberfläche bedeckende Haut (bestehend aus Subkutis, Korium und Epidermis) verstanden, zum anderen das die Hohlorgane und Körperhöhlen auskleidende Gewebe, einschließlich der Epithelien der Gebärmutter und des Mundes.

"Entzündlich verändert" bedeutet im Rahmen der vorliegenden Erfindung "von einer akuten oder chronischen Entzündung betroffen". Die Entzündung kann durch biologische (z. B. Krankheitserreger, Autoimmunreaktionen, TNF), chemische (z. B. Gifte, Reizstoffe) oder physikalische (z. B: UV-Strahlung, osmotische Veränderungen, mechanische Beanspruchung, Hitzestress) Noxen oder Stressoren bedingt sein.

Eine akute Entzündung ist durch plötzliches Auftreten mit raschem, oft heftigem Verlauf über Stunden oder Tage gekennzeichnet.

Die Kardinalsymptome einer akuten Entzündung sind Rubor (Rötung durch Vasodilatation), Tumor (Gewebsschwellung durch entzündliches Exsudat), Calor (Erwärmung aufgrund der vermehrten Gewebsdurchblutung), Dolor (Schmerz durch Nervenreizung) sowie Functio leasa (gestörte Funktion).

Die verschiedenen Phasen einer akuten Entzündung werden durch folgende Mediatoren gesteuert:
a) Zelluläre Mediatoren: biogene vasoaktive Amine (Histamin und Serotonin), Arachidonsäurederivate (Leukotriene, Prostaglandine, Prostazyklin, Thromboxan A2), plättchenaktivierender Faktor (PAF), Zytokine (Interleukine, TNF-a, interferone), NO.
b) Plasmamediatoren: Komplementsystem, Gerinnungs- und fibrinolytisches System, Kallikrein-Kinin-System

Die bekanntesten Formen der akuten Entzündung sind die exsudative Entzündung, seröse Entzündung, fibrinöse Entzündung, eitrige Entzündung, hämorrhagische Entzündung, nekrotisierende und ulzerierende Entzündung, gangränöse Entzündung sowie die akute lymphozytäre Entzündung.

Typisch für eine chronische Entzündung ist hingegen ein langer Verlauf (Wochen, Monate oder Jahre) mit häufig schleichendem Beginn und entwickelnder Symptomatik, insbesondere eine Persistenz der Schädigung.

Eine mithilfe einer erfindungsgemäßen Zubereitung bevorzugt zu behandelnde entzündliche Erkrankung ist die Paradontose. Paradontose ist eine Infektionskrankheit, die in den meisten Fällen hervorgerufen wird durch die Bakterien *Porphyramonas gingivalis, Bacteroides forsythus* und *Actinobacillus actinomycetemcomitans.* Das Vorhandensein der Bakterien ist eine notwendige, aber nicht ausreichende Vorbedingung für das Auftreten der Krankheit. Die kontinuierliche Ausschüttung von schädlichen Substanzen, vor allem Lipopolysacchariden, durch die Bakterien aktiviert das Immunsystem des Wirtes und löst die Entlassung von inflammatorischen Mediatoren und MMPs (Matrix-Metallo-Proteasen) durch die Monocyten aus. Proinflammatorische Cytokine wie IL-1β und TNF-α aktivieren wiederum die Fibroblasten des umgebenden Gewebes, die ihrerseits die Ausschüttung von MMPs verstärken. Aktivierte Makrophagen und Fibroblasten verringern zudem die Expression von TIMPs. Die Folge ist eine Zunahme der Nettoaktivität von MMPs und die Zerstörung des umgebenden Gewebes.

Im Anfangsstadium der Paradontose entsteht durch die MMP-vermittelte Auflösung kleiner Mengen des verbindenden Epithelgewebes zwischen Zahnfleisch und der Zahnwurzeloberfläche eine Tasche, die den Bakterien Zugang zu dem tiefer liegenden Schichten verschafft und somit das Fortschreiten der Krankheit erlaubt. Die Verringerung der Zerstörung der extrazellulären Matrix ist daher ein vielversprechender Ansatz zur Behandlung und Prophylaxe von Paradontose.

Die dem epithelialen Deckgewebe mit Hilfe der erfindungsgemäßen Zubereitung zugeführten Nukleinsäuren sorgen in dem epithelialen Deckgewebe für eine Suppression der überschießenden Immunantwort und dadurch für ein geregeltes Gleichgewicht zwischen Auf- und Abbau von Kollagen.

Die erfindungsgemäße Zubereitung ist außerdem zur Prophylaxe und Behandlung verschiedener anderer Erkrankungen oder unerwünschter Zustände geeignet, insbesondere entzündlich bedingter Alterungsprozesse, Psoriasis, atopisches Ekzern, "trockene Haut", Alopecia arreata, Vitiligo, bullöse Erkrankungen, Abstoßungsreaktionen (graft-versus-host Reaktionen), UV-bedingte Hautentzündungen sowie Funktionsstörungen der epidermalen Barriere, die auf S. 2 der WO 98/32444 aufgezählt sind, auf die hiermit in vollem Umfag Bezug genommen wird.

Im Gegensatz zur Steroidtherapie ist beim Einsatz der erfindungsgemäßen Zubereitung nicht mit unerwünschten Wirkungen zu rechnen, da CpGs natürlich vor kommende DNA-Motive darstellen.

Die erfindungsgemäß einsetzbaren Nukleinsäuren auf der Basis nicht-methytierter CpG-Motive können in dem Fachmann bekannter Weise chemisch synthetisiert oder aus biologischen Quellen, insbesondere aus Bakterien, gewonnen werden.

Die Wirksamkeit von Nukleinsäuren in Formulierungen zur Anwendung insbesondere auf der Haut hängt von der Verfügbarkeit der Nukleinsäuren in den lebenden Zellen der Haut ab. Das Eindringen eines Makromoleküls durch das *Stratum Corneum* (natürliche Barriere der Haut) in die Haut ist nicht immer gewährleistet. In Liposomen verpackte Nukleinsäuren können jedoch das *Stratum Corneum* von Hautmodellen penetrieren. Erfindungsgemäß bevorzugte Zubereitungen sind daher solche, die die erfindungsgemäß einsetzbaren Nukleinsäuren auf der Basis nicht-methylierter CpG-Motive in Liposomen verpackt enthalten.

Besonders bevorzugt erfolgt die Herstellung geeigneter Liposomen wie in der DE-A-197 40 092 beschrieben, auf die hiermit in vollem Umfang Bezug genommen wird.

Ein weiterer Gegenstand der vorliegenden Erfindung ist die Verwendung von Nukleinsäuren auf der Basis nicht-methylierter CpG-Motive zur Prophylaxe und/oder Behandlung epithelialen Deckgewebes, insbesondere zur Prophylaxe und/oder Behandlung entzündlich veränderten epithelialen Deckgewebes.

Ein weiterer Gegenstand der vorliegenden Erfindung ist ein Verfahren zur Herstellung einer kosmetischen oder pharmazeutischen Zubereitung, insbesondere zur Prophylaxe und/oder Behandlung entzündlich veränderten epithelialen Deckgewebes, dadurch gekennzeichnet, daß man Nukleinsäuren auf der Basis nicht-methylierter CpG-Motive, wie für die erfindungsgemäßen Zubereitungen beschrieben, mit kosmetisch und pharmakologisch geeigneten und verträglichen Trägern vermischt.

Weitere Gegenstände der vorliegenden Erfindung sind Wäscheweichspüler (Fabric Softener), Handwaschmittel, Körper und Haarpflegemittel, Haarfärbemittel oder Handgeschirrspülmittel, die Nukleinsäuren auf der Basis nicht-methylierter CpG-Motive, wie für die erfindungsgemäßen Zubereitungen beschrieben, umfassen.

Die Nukleinsäuren auf der Basis nicht-methylierter CpG-Motive werden im Sinne der vorliegenden Erfindung vorzugsweise als Komponente in eine kosmetische oder pharmazeutische Zubereitung oder in Wäscheweichspüler, Handwaschmittel, Handgeschirrspülmittel oder Körperpflegemittel eingebracht bzw. eingearbeitet.

Je nach Art der Formulierung können die erfindungsgemäßen pharmazeutischen Zubereitungen mindestens einen weiteren Hilfs- oder Zusatzstoff, wie z. B. Öle, Schutzkolloide, Weichmacher, Antioxidantien und/oder Emulgatoren enthalten.

Im Falle einer Dispersion, insbesondere im Falle einer Suspension oder Emulsion, ist es vorteilhaft, zusätzlich ein physiologisch verträgliches Öl wie beispielsweise Sesamöl, Maiskeimöl, Baumwollsaatöl, Sojabohnenöl oder Erdnußöl, Ester mittelkettiger pflanzlicher Fettsäuren oder Fischöle wie beispielsweise Makrelen-, Sprotten- oder Lachsöl zu verwenden.

Zur Erhöhung der Stabilität des Wirkstoffes gegen oxidativen Abbau ist es vorteilhaft, Stabilisatoren wie a- Tocopherol, t- Butylhydroxy- toluol, t- Butylhydroxyanisol, Ascorbinsäure oder Ethoxyquine zuzusetzen.

Die Dosierung und Anwendungsdauer der erfindungsgemäß einsetzbaren peptidbasierten Inhibitoren kann durch den Fachmann in geeigneter Weise angepaßt und variiert werden.

Die erfindungsgemäßen Wäscheweichspüler, Handwaschmittel und Handgeschirrspülmittel sowie die kosmetischen Zubereitungen, Körper- und Haarpflegemittel sowie Haarfärbemittel, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparate, Puder oder Salben können - je nach Art der Formulierung - als Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsutfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat in Betracht.

Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z. B. Squalan, Squalen oder Dialkylcyclohexane.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkyl- und/oder Alkenylmono- und -oligoglycoside mit 8 bis 22 Kohlenstoffatomen im Alk(en)ylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar.

Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403), Polyglyceryl Dimerate Isostearate sowie deren Gemische.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole^{®} von Goodrich oder Synthalene^{®} von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalzpolymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ lsobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrytat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/ Dimethylaminoethylmethadrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

Typische Beispiele für Fette sind Glyceride, als Wachse kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel, die gegebenenfalls den erfindungsgemäßen Kosmetika zugesetzt werden, sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Auch Enzyminhibitoren können den erfindungsgemäßen Kosmetika zugesetzt werden. Geeignete Enzyminhibitoren sind beispielsweise Esteraseinhibitoren. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw - phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Apfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallytpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und -Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
(a) adstringierende Wirkstoffe,
(b) Ölkomponenten,
(c) nichtionische Emulgatoren,
(d) Coemulgatoren,
(e) Konsistenzgeber,
(f) Hilfsstoffe wie z. B. Verdicker oder Komplexierungsmittel und/oder
(g) nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2-Aluminiumhydroxyallantoinat, Aluminiumchloridtartrat, Aluminium-Zirkonium-Trichiorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als Antischuppenmittel können Climbazol, Octopirox und Zinkpyrithion eingesetzt werden.

Gebräuchliche Filmbildner sind beispielsweise Chitosan, mikrokristallines Chitosan, quaterniertes Chitosan, Polyvinylpyrrolidon, Vinylpyrrolidon-VinylacetatCopolymerisate, Polymere der Acrylsäurereihe, quaternäre Cellulose-Derivate, Kollagen, Hyaluronsäure bzw, deren Salze und ähnliche Verbindungen.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der EP 0693471 B1 beschrieben;
- 4-Aminobenzoesäurederivate, vorzugsweise 4-Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
- Propan-1,3-dione, wie z.B. 1-(4-tert.Butylphenyl)-3-4'methoxyphenyl)propan-1,3-dion;
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsutfonsäure und 2-Mothyl-5-(2-oxo-3-bornyliden)-sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Joumal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D.L-Carnosin, D-Carnosin, L-Carnosin und deren Derivate (z.B. Anserin), Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren(z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Außerdem können erfindungsgemäß Verbindungen zur Unterdrückung oder Minderung von Hautstörungen zugesetzt werden, die durch UV-Strahlung induziert werden, insbesondere Aktivatoren von Peroxisom-Proliferator-Aktivierten Rezeptoren (PPAR-Aktivatoren), wie in der WO 02/38150 beschrieben, auf die hiermit in vollem Umfang Bezug genommen wird.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandioi.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-tsomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Zu den erfindungsgemäßen Körperpflegemitteln zählen auch Zahnpflegemittel und allgemein Mittel zur Pflege der Mundhygiene (Oral Care Produkte).

Zahnpasten enthalten z. B. typischerweise:
- Putz- und Polierkörper wie z.B. Kreide, Kieselsäuren, Aluminiumhydroxid, Aluminiumsilikate, Calciumpyrophosphat, Dicalciumphosphat, unlösliches Natriummetaphosphat oder Kunstharzpulver;
- Feuchthaltemittel wie z.B. Glycerin, 1,2-Propylenglycol, Sorbit, Xylit und Polyethylenglycole
- Bindemittel und Konsistenzregler, z.B. natürliche und synthetische wasserlösliche Polymere und wasserlösliche Derivate von Naturstoffen, z.B. Celluloseether, Schichtsilikate, feinteilige Kieselsäuren (Aerogel-Kieselsäuren, pyrogene Kieselsäuren)
- Aromen, z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen
- Süßstoffe wie z.B. Saccharin-Natrium, Natrium-cyclamat, Aspartame, Acesulfan K, Steviosid, Monellin, Glycyrrhicin, Dulcin, Lactose, Maltose oder Fructose
- Konservierungsmittel und antimikrobielle Stoffe wie z.B. p-Hydroxybenzoesäureester, Natriumsorbat, Triclosan, Hexachlorphen, Phenylsalicylsäureeter, Thymol usw.
- Pigmente wie z.B. Titandioxid oder Pigmentfarbstoffe zur Erzeugung farbiger Streifen
- Puffersubstanzen z.B. primäre, sekundäre oder tertiäre Alkaliphosphate, Citronen-säure/Na-Citrat
- wundheilende und entzündungshemmende Wirkstoffe, z.B. Allantoin, Harnstoff, Azulen, Panthenol, Acetylsalicylsäure-Derivate, Pflanzenextrakte, Vitamine, z.B. Retinol oder Tocopherol.

Der Gesamtanteil der Hilfs- und Zusatzstoffe kann 1 bis 50, vorzugsweise 5 bis 40 Gew.-% - bezogen auf die Mittel - betragen. Die Herstellung der Kosmetika und Körperpflegemittel kann durch übliche Kalt - oder Heißprozesse erfolgen; vorzugsweise arbeitet man nach der Phaseninversionstemperatur-Methode.

Die folgenden Beispiele beschreiben die Erfindung, ohne sie jedoch darauf einzuschränken:

### Beispiele 1 bis 9

In in vitro Modellen mit primären Keratinozyten und einer Keratinozytenlinie (Ha-CaT) wurde sowohl die basale als auch die induzierte Freisetzung von proinflammatorischen Zytokinen gemessen.

Entzündung wurde u. a. durch Bestrahlung mit 150mJ/cm² UVB-Licht (Waldmann 3003 K light cabin, Waldmann, Villingen-Schwenningen, Germany), durch 20ng/ml TNFa und durch anisosmolare Bedingungen (-100mM NaCl) induziert.

Die Anwendung von UVB und TNFa zur in vitro Stimulierung von Keratinozyten ist in der dermatologischen Forschung gut etabliert (Kippenberger S, Loitsch SM, Grundmann-Kollmann M, Simon S, Dang TA, Hardt-Weinelt K, Kaufmann R, Bernd A. Activators of peroxisome proliferator-activated receptors protect human skin from ultraviolet-B-light-induced inflammation. J Invest Dermatol; 117:1430-1436, 2001).

Die Wirksamkeit von anisomolaren Bedingungen ist an Lungenepithelzellen ebenfalls gezeigt worden (Loitsch SM, von Mallinckrodt G, Kippenberger S, Steinhilber D, Wagner TO, Bargon J. Reactive oxygen intermediates are involved in 11-8 production induced by hyperosmotic stress in human bronchial epithelial cells. Biochem Biophys Res Commun; 276:571-578, 2000; Hashimoto S, Matsumoto K, Gon Y, Nakayama T, Takeshita I, Horie T. Hyperosmolarity-induced interleukin-8 expression in human bronchial epithelial cells through p38 mitogen-activated protein kinase. Am J Respir Crit Care Med; 159:634-640, 1999).

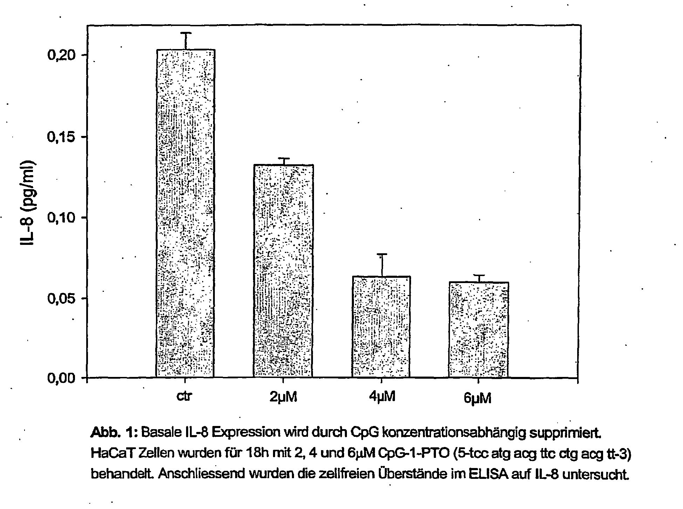

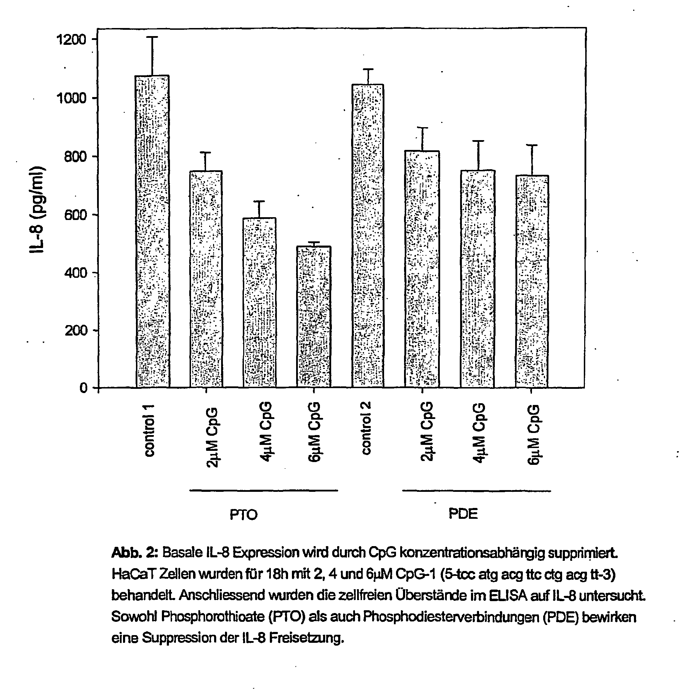

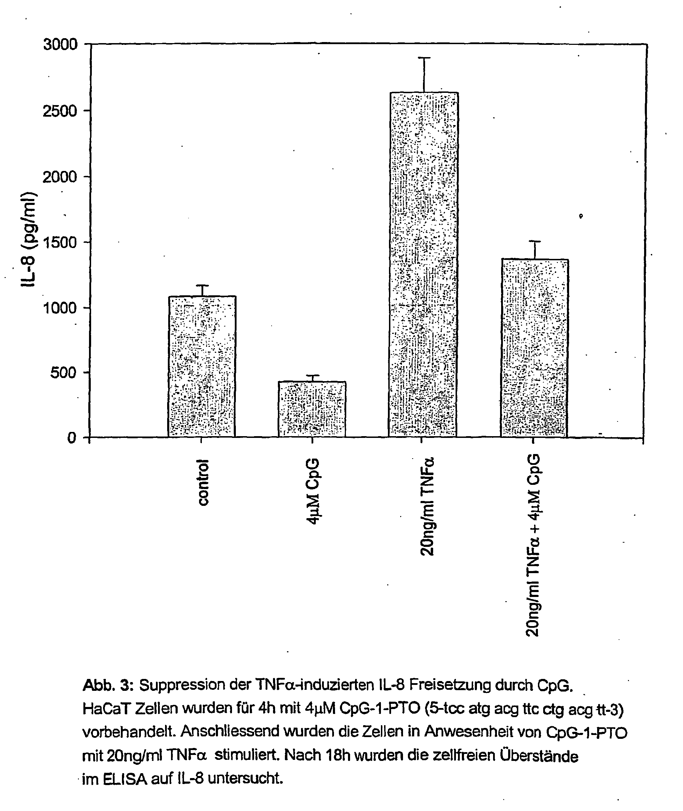

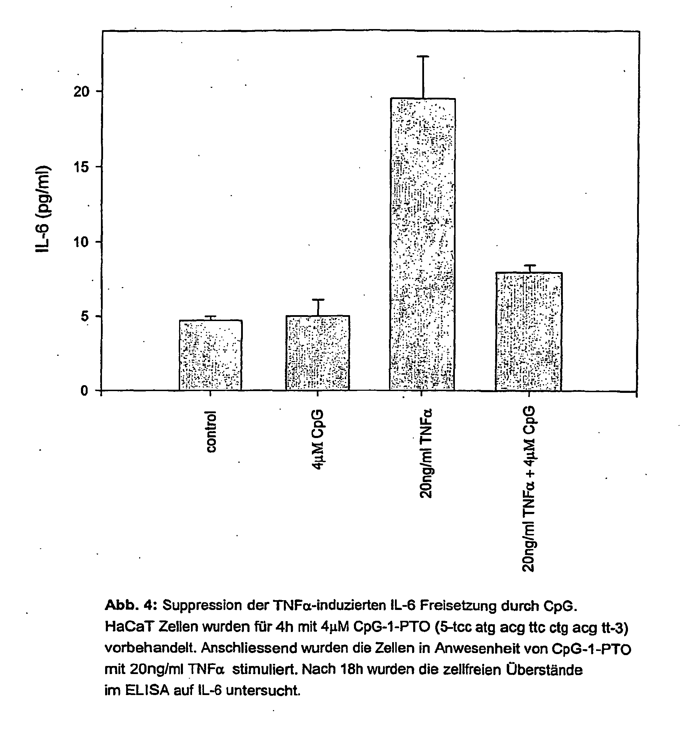

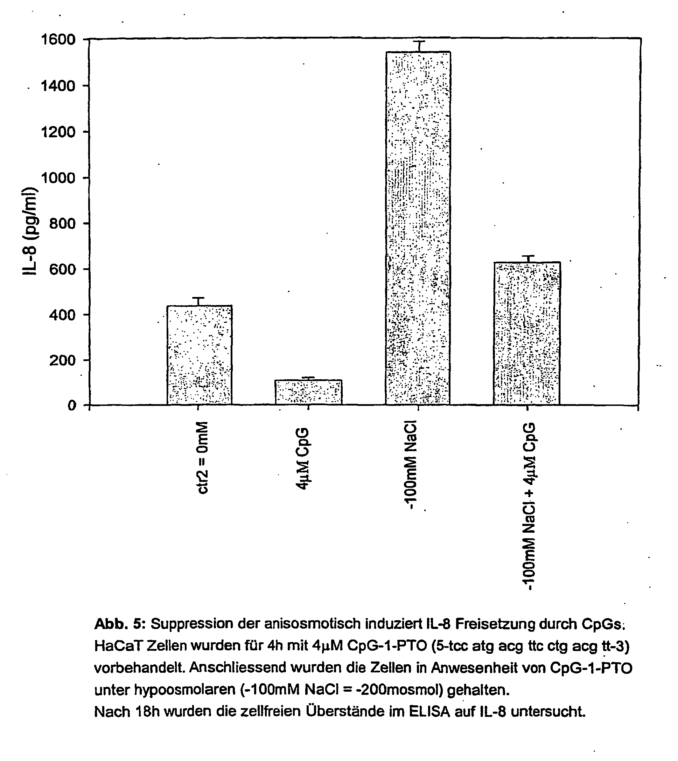

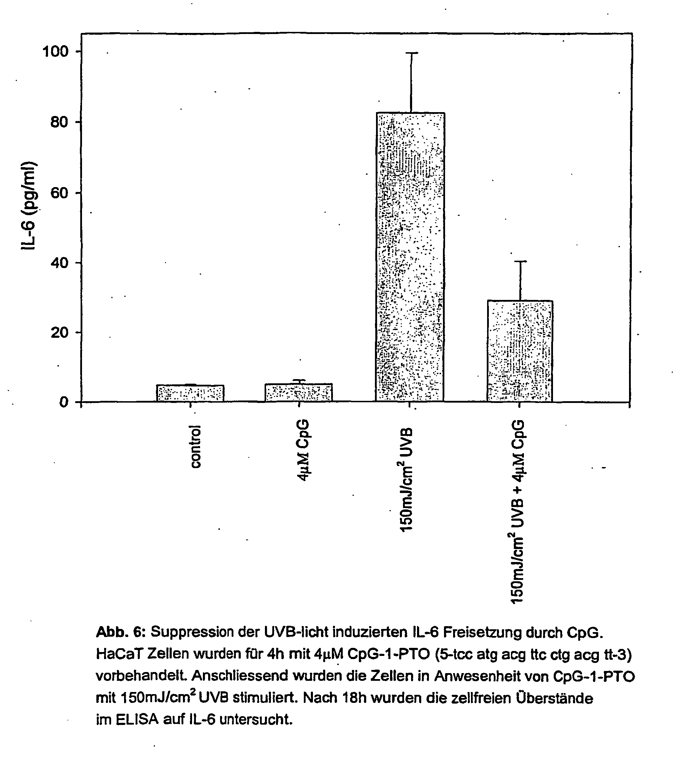

Die jeweils verwendete Zellspezies und der Versuchsaufbau sind auf den Abbildungen 1-6, die die Beispiele 1-6 darstellen, vermerkt.

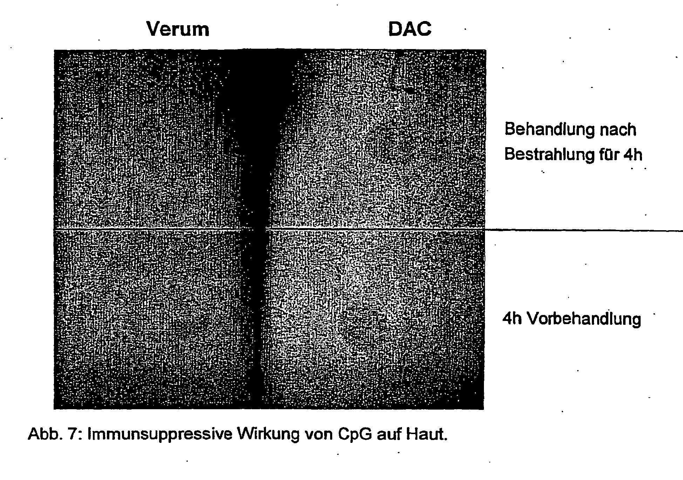

Neben den *in vitro* Daten, die eine Immunsuppression auf Hautzellen zeigen, hat sich diese Wirkung auch in einem *in vivo* Selbstversuch bestätigt (s. Abb. 7). Hierbei wurde die Sequenz von CpG-1 (5'-TCC ATG ACG TTC CTG ACG TT-3') als Phosphorothioat 1,4%ig in DAC-Basiscreme eingearbeitet. In Ansatz A) wurde die wirkstoffhaltige Creme für 4h auf die unbehandelte Haut aufgetragen und danach entfernt. Anschliessend wurde die so vorbehandelte Haut mit 90mJ/cm² UVB-Licht bestrahlt (Saalmann Multitester, Saalmann, Herford, Germany). In Ansatz B) wurde die unbehandelte Haut zunächst mit 90mJ/cm² UVB bestrahlt und danach für 4h mit der CpG-haltigen Salbe behandelt. In den Kontrollen wurde die Haut mit Placebo (wirkstofffreie DAC-Grundlage) behandelt. Es zeigte sich in den Placebokontrollen, dass UVB-Licht zu deutlich sichtbaren Erythemen führt. Sowohl die Behandlung *vor* bzw. *nach* Applikation der UVB-Noxe mit CpG-hahiger Creme führte zu deutlich schwächeren UVB-Erythemen. Dies belegt die überraschende immunsuppressive Wirkung von CpG auf das Hautorgan.

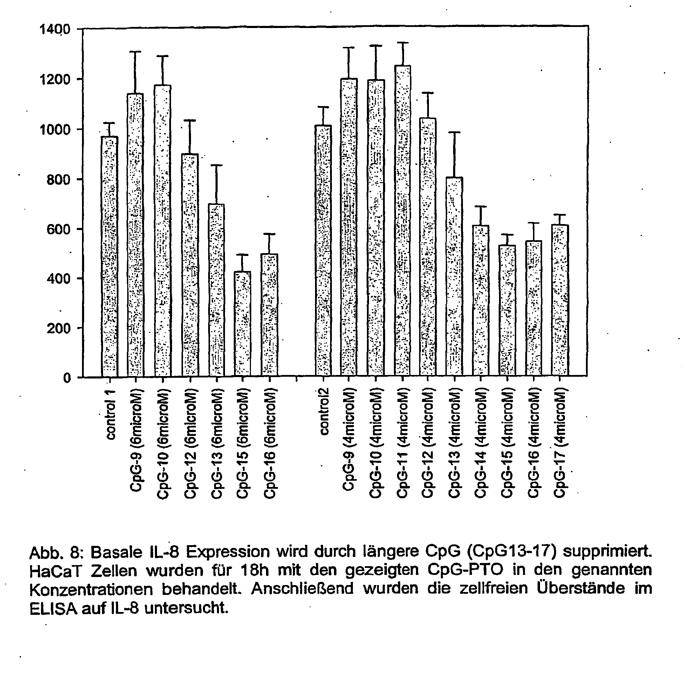

Weiter konnte festgestellt werden, daß die Länge der Oligonukleotide bedeutsam für die Wirksamkeit ist (s. Abb. 8). Getestet wurde CpG-Oligonukleotide, die durch proximale und distale Deletionen von CpG-1 hervorgegangen sind, hier die Sequenzen im Detail:

| | | |
|---|---|---|
| 1. | CpG-1-PTO: | 5'-TCC ATG ACG TTC CTG ACG TT-3' |
| 2. | CpG-9-PTO: | 5'-G ACG TT-3' . |
| 3. | CpG-10-PTO: | 5'-TG ACG TTC-3' |
| 4. | CpG-11-PTO: | 5'-ATG ACG TTC C-3' |
| 5. | CpG-12-PTO: | 5'-C ATG ACG TTC CT-3' |
| 6. | CpG-13-PTO: | 5'-CC ATG ACG TTC CTG-3' |
| 7. | CpG-14-PTO: | 5'-TCC ATG ACG TTC CTG A-3' |
| 8. | CpG-14A-PTO: | 5'-TCC TCA ACG TTC CTG A-3' |
| 9. | CpG-14B-PTO: | 5'-TCC GCA ACG TTC CTG A-3' |
| 10. | **CpG-14C-PTO:** | **5'-TCC TCG ACG TCC CTG A-3'** |
| 11. | CpG-14D-PTO: | 5'-TCC TCA GCG CTC CTG A-3' |
| 12. | CpG-14E-PTO: | 5'-TCC TCA ACG CTC CTG A-3' |
| 13. | CpG-14F-PTO: | 5'-TCC TCA TCG ATC CTG A-3' |
| 14. | CpG-14G-PTO: | 5'-TCC TCT TCG AAC CTG A-3' |
| 15. | CpG-15-PTO: | 5'-TCC ATG ACG TTC CTG AC-3' |
| 16. | CpG-16-PTO: | 5'- TCC ATG ACG TTC CTG ACG-3' |
| 17. | CpG-17-PTO: | 5'- TCC ATG ACG TTC CTG ACG T-3' |

Es konnte gezeigt werden, dass eine besonders starke immunsuppressive Wir kung ab einer Gesamtlänge von ≥ 14 Basen detektierbar ist.

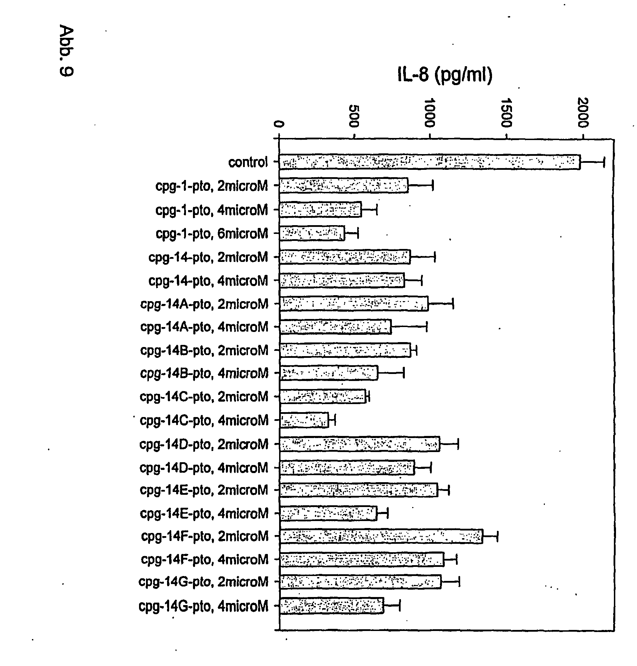

Zu den erfindungsgemäß besonders bevorzugten Sequenzen gehört CpG-14C-PTO (s. Abb. 9).

## Patentansprüche

1. Kosmetische oder pharmazeutische Zubereitung zur Prophylaxe und/oder Behandlung epithelialen Deckgewebes, die Nukleinsäuren auf der Basis nicht-methylierter CpG-Motive enthält, **dadurch gekennzeichnet, dass** die Nukleinsäuren eine Sequenz umfassen, die ausgewählt ist aus
5'-TCC ATG ACG TTC CTG ACG TT-3' (Seq-ID No.1)
5'-G ACG TT-3' (Seq-ID No.2)
5'-TG ACG TTC-3' (Seq-ID No.3)
5'-ATG ACG TTC C-3' (Seq-ID No.4)
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No.14)
5'-TCC ATG ACG TTC CTG AC-3' (Seq-ID No.15)
5' - TCC ATG ACG TTC CTG ACG-3' (Seq-ID No.16)
5'- TCC ATG ACG TTC CTG ACG T-3' (Seq-ID No.17)

2. Zubereitung nach Anspruch 1, **dadurch gekennzeichnet, dass** die enthaltenen Nukleinsäuren ausgewählt sind aus
5'-TCC ATG ACG TTC CTG ACG TT-3' (Seq-ID No.1)
5'-G ACG TT-3' (Seq-ID No.2)
5'-TG ACG TTC-3' (Seq-ID No.3)
5'-ATG ACG TTC C-3' (Seq-ID No.4
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No.14)
5'-TCC ATG ACG TTC CTG AC-3' (Seq-ID No.15)
5'- TCC ATG ACG TTC CTG ACG-3' (Seq-ID No.16)
5'- TCC ATG ACG TTC CTG ACG T-3' (Seq-ID No.17)
insbesondere 5'-TCC TCG ACG TCC CTG A-3' (Seq-ID No.10).

3. Zubereitung nach Anspruch 1 oder 2 zur Prophylaxe und/oder Behandlung entzündlich veränderten epithelialen Deckgewebes.

4. Zubereitung nach einem der vorstehenden Ansprüche zur Prophylaxe und/oder Behandlung von entzündlichen Veränderungen, die ausgewählt sind unter chronischen oder akuten Entzündungen, insbesondere unter exsudativen Entzündungen, serösen Entzündungen, fibrinösen Entzündungen, eitrigen Entzündungen, hämorrhagischen Entzündungen, nekrotisierenden und ulzerierenden Entzündungen, gangränösen Entzündungen sowie akuten lymphozytären Entzündungen.

5. Zubereitung nach einem der vorhergehenden Ansprüche, zur Prophylaxe und/oder Behandlung von entzündlichen Veränderungen, die durch Noxen oder Stressoren bedingt sind, die ausgewählt sind unter:
a) biologischen Noxen oder Stressoren, insbesondere Krankheitserreger, Autoimmunreaktionen, TNF,
b) chemischen Noxen oder Stressoren, insbesondere Gifte, Reizstoffe und
c) physikalischen Noxen oder Stressoren, insbesondere UV-Strahlung, osmotische Veränderungen, mechanische Beanspruchung, Hitzestress.

6. Zubereitung nach einem der vorhergehenden Ansprüche zur Prophylaxe und/oder Behandlung von entzündlichen Veränderungen, die ausgewählt sind unter entzündlich bedingten Alterungsprozessen, Psoriasis, atopisches Ekzem, "trockene Haut", Alopecia arreata, Vitiligo, bullösen Erkrankungen, Abstoßungsreaktionen, UV-bedingten Hautentzündungen und Parodontose.

7. Zubereitung nach einem der vorhergehenden Ansprüche, **dadurch gekennzeichnet, dass** die Nukleinsäuren auf der Basis nicht-methylierter CpG-Motive eine Länge von 6 bis 40, insbesondere 14 bis 40, vorzugsweise 14 bis 30 bevorzugt 14 bis 25 und ganz besonders bevorzugt von 14 bis 20 Nukleotiden aufweisen.

8. Zubereitung nach einem der vorhergehenden Ansprüche, **dadurch gekennzeichnet, dass** die Nukleinsäuren auf der Basis nicht-methylierter CpG-Motive vollständig oder teilweise chemisch modifiziert sind.

9. Zubereitung nach Anspruch 8, **dadurch gekennzeichnet, dass** die chemische Modifikation ausgewählt ist unter
a) Veränderung der Internucleosidbrücken, insbesondere Austausch von Phosphodiestern gegen Methylphosphonate, Phosphoramidate, Phosphorothioate oder Hydroxylamine;
b) Veränderung der Zuckerkomponenten, insbesondere Austausch der Ribose gegen diverse Hexo- bzw. Pentopyranosen oder 3'-5'-carbocyclisch verbrückte Derivate der 2'-Deoxyribose; oder
c) Austausch des Strangrückgrats, insbesondere Austausch der Polyesterketten auf Basis von Zucker-Phosphat-Einheiten gegen Carboxamidketten auf Basis von Aminosäurederivaten, wie N-(2-Aminoethyl)-glycin-Einheiten.

10. Zubereitung nach einem der vorhergehenden Ansprüche, **dadurch gekennzeichnet, dass** sie die Nukleinsäuren auf der Basis nicht-methylierter CpG-Motive in Liposomen verpackt enthält.

11. Verfahren zur Herstellung einer kosmetischen oder pharmazeutischen Zubereitung zur Prophylaxe und/oder Behandlung epithelialen Deckgewebes, insbesondere zur Prophylaxe und/oder Behandlung entzündlich veränderten epithelialen Deckgewebes, **dadurch gekennzeichnet, dass** man Nukleinsäuren auf der Basis nicht-methylierter CpG-Motive, wie in den Ansprüchen 1 bis 10 beschrieben, mit kosmetisch und pharmakologisch geeigneten und verträglichen Trägern vermischt.

12. Wäscheweichspüler, Handwaschmittel, Körper- und Haarpflegemittel, Haarfärbemittel oder Handgeschirrspülmittel, umfassend Nukleinsäuren auf der Basis nicht-methylierter CpG-Motive, wie in den Ansprüchen 1 und 2 beschrieben.

## Claims

1. Cosmetic or pharmaceutical preparation for the prophylaxis and/or treatment of epithelial integument, which comprises nucleic acids based on non-methylated CpG motifs, **characterized in that** the nucleic acids include a sequence which is selected from
5'-TCC ATG ACG TTC CTG ACG TT-3'(Seq-ID No.1)
5'-G ACG TT-3' (Seq-ID No.2)
5'-TG ACG TTC-3' (Seq-ID No.3)
5'-ATG ACG TTC C-3'(Seq-ID No.4)
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No.14)
5'-TCC ATG ACG TTC CTG AC-3' (Seq-ID No. 15)
5'- TCC ATG ACG TTC CTG ACG-3' (Seq-ID No.16)
5'- TCC ATG ACG TTC CTG ACG T-3' (Seq-ID No.17)

2. Preparation according to Claim 1, **characterized in that** the included nucleic acids are selected from
5'-TCC ATG ACG TTC CTG ACG TT-3' (Seq-tD No.1)
5'-G ACG TT-3' (Seq-ID No.2)
5'-TG ACG TTC-3' (Seq-ID No.3)
5'-ATG ACG TTC C-3' (Seq-ID No.4
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No.14)
5'-TCC ATG ACG TTC CTG AC-3' (Seq-ID No. 15)
5'- TCC ATG ACG TTC CTG ACG-3' (Seq-ID No.16)
5'- TCC ATG ACG TTC CTG ACG T-3' (Seq-ID No.17)
in particular 5'-TCC TCG ACG TCC CTG A-3' (Seq-ID No.10).

3. Preparation according to Claim 1 for the prophylaxis and/or treatment of epithelial integument with inflammatory changes.

4. Preparation according to any of the preceding claims for the prophylaxis and/or treatment of inflammatory changes selected from chronic or acute inflammations, in particular from exudative inflammations, serous inflammations, fibrinous inflammations, purulent inflammations, hemorrhagic inflammations, necrotizing and ulcerating inflammations, gangrenous inflammations and acute lymphocytic inflammations.

5. Preparation according to any of the preceding claims, for the prophylaxis and/or treatment of inflammatory changes caused by noxae or stressors which are selected from:
a) biological noxae or stressors, especially pathogens, autoimmune reactions, TNF,
b) chemical noxae or stressors, especially poisons, irritants and
c) physical noxae or stressors, in particular UV radiation, osmotic changes, mechanical stress, thermal stress.

6. Preparation according to any of the preceding claims for the prophylaxis and/or treatment of inflammatory changes which are selected from aging processes related to inflammation, psoriasis, atopic eczema, "dry skin", alopecia areata, vitiligo, bullous disorders, rejection reactions, UV-related cutaneous inflammations and parodontosis.

7. Preparation according to any of the preceding claims, **characterized in that** the nucleic acids based on non-methylated CpG motifs have a length of from 6 to 40, in particular 14 to 40, preferably 14 to 30, more preferably 14 to 25 and very particularly preferably from 14 to 20, nucleotides.

8. Preparation according to any of the preceding claims, **characterized in that** the nucleic acids based on non-methylated CpG motifs are completely or partially chemically modified.

9. Preparation according to Claim 8, **characterized in that** the chemical modification is selected from
a) modification of the internucleoside bridges, in particular replacement of phosphodiesters by methylphosphonates, phosphoramidates, phosphorothioates or hydroxylamines;
b) modification of the sugar components, in particular replacement of ribose by various hexo- or pentopyranoses or 3'-5'-carbocyclically bridged derivatives of 2'-deoxyribose; or
c) replacement of strand backbone, in particular replacement of the polyester chains based on sugar-phosphate units by carboxamide chains based on amino acid derivatives such as N-(2-aminoethyl)glycine units.

10. Preparation according to any of the preceding claims, **characterized in that** it comprises the nucleic acids based on non-methylated CpG motifs packaged in liposomes.

11. Process for producing a cosmetic or pharmaceutical preparation for the prophylaxis and/or treatment of epithelial integument, in particular for the prophylaxis and/or treatment of epithelial integument with inflammatory changes, **characterized in that** nucleic acids based on non-methylated CpG motifs as described in claims 1 to 10 are mixed with cosmetically and pharmacologically suitable and acceptable carriers.

12. Fabric softener, handwashing composition, body- and hair-care composition, hair-coloring composition or manual dishwashing composition including nucleic acids based on non-methylated CpG motifs as described in claims 1 and 2.

## Revendications

1. Préparation cosmétique ou pharmaceutique pour la prophylaxie et/ou le traitement de l'épithélium, qui contient des acides nucléiques à base motifs CpG non méthylés, **caractérisée en ce que** les acides nucléiques comprennent une séquence qui est choisie parmi
5'-TCC ATG ACG TTC CTG ACG TT-3' (Seq-ID No.1)
5'-G ACG TT-3' (Seq-ID No.2)
5'-TG ACG TTC-3' (Seq-ID No.3)
5'-ATG ACG TTC C-3' (Seq-ID No.4)
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No. 14)
5'-TCC ATG ACG TTC CTG AC-3' (Seq-ID No. 15)
5'- TCC ATG ACG TTC CTG ACG-3' (Seq-ID No.16)
5'- TCC ATG ACG TTC CTG ACG T-3' (Seq-ID No.17)

2. Préparation selon la revendication 1, **caractérisée en ce que** les acides nucléiques contenus sont choisis parmi
5'-TCC ATG ACG TTC CTG ACG TT-3' (Seq-ID No.1)
5'-G ACG TTC-3' (Seq-ID No.2)
5'-TG ACG TTC-3' (Sect-ID No.3)
5'-ATG ACG TTC C-3' (Seq-ID No.4
5'-C ATG ACG TTC CT-3' (Seq-ID No.5)
5'-CC ATG ACG TTC CTG-3' (Seq-ID No.6)
5'-TCC ATG ACG TTC CTG A-3' (Seq-ID No.7)
5'-TCC TCA ACG TTC CTG A-3' (Seq-ID No.8)
5'-TCC GCA ACG TTC CTG A-3' (Seq-ID No.9)
5'-TCC TCG ACG TCC CTG A-3' (Seq-ID No.10)
5'-TCC TCA GCG CTC CTG A-3' (Seq-ID No.11)
5'-TCC TCA ACG CTC CTG A-3' (Seq-ID No.12)
5'-TCC TCA TCG ATC CTG A-3' (Seq-ID No.13)
5'-TCC TCT TCG AAC CTG A-3' (Seq-ID No. 14)
5'-TCC ATG ACG TTC CTG AC-3' (Seq-ID No. 15)
5'- TCC ATG ACG TTC CTG ACG-3' (Seq-ID No.16)
5'- TCC ATG ACG TTC CTG ACG T-3' (Sea-ID No.17)
en particulier 5'-TCC TCG ACG TCC CTG A-3' (Seq-ID No.10)_{.}

3. Préparation selon la revendication 1 ou 2 pour la prophylaxie et/ou le traitement d'épithélium à modification inflammatoire.

4. Préparation selon l'une quelconque des revendications précédentes pour la prophylaxie et/ou le traitement de modifications inflammatoires, qui sont choisies parmi les inflammations chroniques ou aiguës, en particulier parmi les inflammations exsudatives, les inflammations séreuses, les inflammations fibrineuses, les inflammations suppuratives, les inflammations hémorragiques, les inflammations nécrosantes et ulcératives, les inflammations gangréneuses, ainsi que les inflammations lymphocytaires aiguës.

5. Préparation selon l'une quelconque des revendications précédentes, pour la prophylaxie et/ou le traitement de modifications inflammatoires, qui sont provoquées par des substances nocives ou des facteurs de stress, qui sont choisies parmi:
a) les substances nocives ou facteurs de stress biologiques, en particulier les agents pathogènes, les réactions auto-immunes, TNF,
b) les substances nocives ou facteurs de stress, chimiques, en particulier les poisons, les substances irritatives et
c) les substances nocives ou facteurs de stress, physiques, en particulier le rayonnement UV, les modifications osmotiques, la sollicitation mécanique, la contrainte thermique.

6. Préparation selon l'une quelconque des revendications précédentes pour la prophylaxie et/ou le traitement de modifications inflammatoires, qui sont choisies parmi les processus de vieillissement provoqués par une inflammation, le psoriasis, l'eczéma atopique, la « peau sèche », la pelade, le vitiligo, les maladies bulleuses, les réactions de rejet, les inflammations cutanées provoquées par les UV et la parodontose.

7. Préparation selon l'une quelconque des revendications précédentes, **caractérisée en ce que** les acides nucléiques à base de motifs CpG non méthylés présentent une longueur de 6 à 40, en particulier de 14 à 40, de préférence de 14 à 30, préférentiellement de 14 à 25, et de manière tout particulièrement préférée de 14 à 20 nucléotides.

8. Préparation selon l'une quelconque des revendications précédentes, **caractérisée en ce que** les acides nucléiques à base de motifs CpG non méthylés sont entièrement ou partiellement modifiés chimiquement.

9. Préparation selon la revendication 8, **caractérisée en ce que**, la modification chimique est choisie parmi
a) la modification des ponts entre nucléosides, en particulier l'échange de phospho-diesters contre des phosphonates de méthyle, des phosphoramidates, des phosphorothioates, ou des hydroxylamines ;
b) la modification des composants glucidiques, en particulier l'échange du ribose contre divers hexapyranoses ou pentapyranoses, ou des dérivés à pontage 3'-5'-carbocycliques du 2'-desoxyribose ; ou
c) l'échange de l'épine dorsale de brin, en particulier l'échange des chaînes polyester à base de motifs glucide-phosphate contre des chaînes carboxamides à base de dérivés d'acides aminés, comme les motifs N-(2-aminoéthyl)-glycine.

10. Préparation selon l'une quelconque des revendications précédentes, **caractérisée en ce qu'**elle contient les acides nucléiques à base de motifs CpG non méthylés, encapsulés dans des liposomes.

11. Procédé pour préparer une préparation cosmétique ou pharmaceutique pour la prophylaxie et/ou le traitement d'épithélium, en particulier pour la prophylaxie et/ou le traitement d'épithélium à modification inflammatoire, **caractérisé en ce que** l'on mélange des acides nucléiques à base de motifs CpG non méthylés, tels que décrits selon les revendications 1 à 10, avec des véhicules cosmétiquement et pharmacologiquement appropriés et compatibles.

12. Produit adoucissant pour le linge, produit pour le lavage à la main, produit de soins pour le corps et les cheveux, teinture capillaire ou produit vaisselle à la main, comprenant des acides nucléiques à base de motifs Cpg non méthylés, tels que décrits selon les revendications 1 et 2.
